# EUROPÄISCHE PATENTSCHRIFT

(11) **EP 2 501 348 B1**
(45) Veröffentlichungstag und Bekanntmachung des Hinweises auf die Patenterteilung: **21.12.2016**
(21) Anmeldenummer: 09768494.8
(22) Anmeldetag: 18.11.2009
(51) Int. Cl.: A61F 9/008, A61F 9/01, H01S 3/109, H01S 3/13

(54) **MATERIALBEARBEITUNGSEINRICHTUNG UND VERFAHREN ZUM BETREIBEN EINER SOLCHEN**
MATERIAL PROCESSING DEVICE AND METHOD FOR OPERATING SUCH A MATERIAL PROCESSING DEVICE
INSTALLATION DE TRAITEMENT DE MATÉRIAU ET PROCÉDÉ DE MISE EN OEUVRE ASSOCIÉ

(43) Veröffentlichungstag der Anmeldung: 26.09.2012
(73) Patentinhaber: WaveLight GmbH, 91058 Erlangen (DE)
(72) Erfinder: WOITTENNEK, Franziska, 01129 Dresden (DE); WÖLFEL, Mathias, 91052 Erlangen (DE); VOGLER, Klaus, 99444 Blankenhain (DE); KITTELMANN, Olaf, 14109 Berlin (DE)
(74) Vertreter: Katérle, Axel
(86) Internationale Anmeldenummer: PCT/EP2009/008209
(87) Internationale Veröffentlichungsnummer: WO 2011/060797

(56) Entgegenhaltungen:
- EP-A1- 1 281 378
- EP-A1- 1 748 312
- EP-A1- 2 109 197
- US-A1- 2004 199 150
- US-A1- 2005 163 174

## Beschreibung

Die Erfindung betrifft eine Einrichtung zur Bearbeitung eines Materials mit einem gepulsten Laserstrahl. Ferner betrifft die Erfindung ein Verfahren zum Betreiben einer solchen Materialbearbeitungseinrichtung.

Grundsätzlich ist die Erfindung im Umfeld von Lasersystemen anwendbar, die zur Bearbeitung beliebiger Materialien dienen. Dementsprechend kann das zu bearbeitende Material tote Materie sein; es kann sich aber auch um lebende Materie handeln, beispielsweise Gewebe eines humanen Auges.

Bei der Bearbeitung von Materialien, insbesondere solcher Materialien, die im sichtbaren Spektralbereich transparent sind, gewinnen sogenannte Femtosekunden-Lasersysteme zunehmende Bedeutung. Dabei handelt es sich um Lasersysteme, die einen gepulsten fokussierten Laserstrahl mit Pulsdauern im Femtosekundenbereich erzeugen. Solche Femtosekunden-Lasersysteme finden Anwendung beispielsweise in der laserchirurgischen Ophthalmologie, wo sie überlicherweise zur Erzeugung von Schnitten (Inzisionen) im kornealen Gewebe oder in anderen Gewebebereichen des humanen Auges eingesetzt werden. Vorteilhaft an Femtosekunden-Lasersystemen ist die Eignung zur Erzeugung an sich beliebiger dreidimensionaler Schnittfiguren.

Im Folgenden sei kurz der prinzipielle Elementarprozess bei der Bearbeitung transparenter Materialien mittels fokussierter Femtosekunden-Laserstrahlung erläutert. Durch die starke Fokussierung des Laserstrahls in das Material und wegen der Transparenz des Materials für die Strahlung kann die Laserleistung in das Innere eingekoppelt werden, ohne dass das durchstrahlte Material (z.B. korneales Gewebe) oberhalb des Fokuspunkts geschädigt wird. Der Vorgang, der im Fokuspunkt stattfindet, wird als Photodisruption bezeichnet. Im Fokuspunkt wird aufgrund der hochintensiven Strahlung die Schwelle für die Entstehung von Mikroplasma überschritten. Es kommt zu einer Verdampfung einer äußerst kleinen Materialkugel von beispielsweise etwa 1 µm Durchmesser. Als Folge entsteht eine Mikroblase mit einem etwas größeren Durchmesser, beispielsweise etwa 5-12 µm, die das umliegende Material voneinander trennt und anschließend komplett in die Umgebung diffundiert. Durch die extrem kurze Einwirkzeit pro Laserpuls kann keine Wärmeleitung zum umliegenden Material stattfinden; sämtliche wirksame Energie sowie Wärme ist nach dem Verschwinden des Plasmas wieder dissipiert.

Bei üblichen Femtosekunden-Lasersystemen ist der Fokuspunkt durch eine Ablenkvorrichtung (Scanner) transversal und auch longitudinal steuerbar. Transversal meint hierbei eine Richtung in einer Ebene, welche orthogonal zur Strahlausbreitungsrichtung liegt. Longitudinal meint dagegen eine Richtung entlang der Ausbreitungsrichtung des Laserstrahls. Setzt man eine entsprechende Anzahl (beispielsweise mehrere tausend) der durch Plasmaentladung entstandenen Hohlräume in der gewünschten Form dreidimensional aneinander, so entsteht der gewünschte Schnitt im Material.

Das obige Verfahren verlangt einen sehr präzisen Fokuspunkt mit hoher Spitzenintensität, um die Schnitte mit der gewünschten Genauigkeit anzubringen. Die Fokussierbarkeit und die Spitzenintensität sind jedoch empfindliche Parameter; bereits vergleichsweise geringfügige Störungen im Ausbreitungsweg des Laserstrahls können die räumliche und zeitliche Qualität des Laserstrahls und damit dessen Fokussierbarkeit und Spitzenintensität beeinträchtigen. Während der Materialbearbeitung ist es deshalb wünschenswert, die zeitliche und räumliche Qualität des Laserstrahls fortlaufend (kontinuierlich oder zumindest mehrmals in zeitlichen Abständen) zu überwachen.

Das Dokument US 2005/0163174 A1 beschreibt eine Vorrichtung und ein Verfahren zur Erzeugung gepulster höher-harmonischer Laserstrahlen. Die Vorrichtung umfasst einen Laser zur Erzeugung eines gepulsten Laserstrahls einer Grundwelle, der innerhalb eines aus zwei Endspiegeln bestehenden optischen Resonators oszilliert. Ein KTP (KTiOPO₄) Kristall ist im Strahlengang des optischen Resonators angeordnet, um einen gepulsten Laserstrahl einer zweiten Harmonischen zu erzeugen. Ebenfalls ist ein Separator-Auslass-Spiegel im Strahlengang des optischen Resonators angeordnet, der den gepulsten Laserstrahl der Grundwelle im Strahlengang des optischen Resonators belässt und den gepulsten Laserstrahl der zweiten Harmonischen aus dem optischen Resonator extrahiert. Der gepulste Laserstrahl der zweiten Harmonischen wird auf ein Werkstück gerichtet. Die Laserleistung des gepulsten Laserstrahls der Grundwelle wird mit einem Messkreis gemessen. Ebenso wird die Laserleistung des gepulsten Laserstrahls der zweiten Harmonischen mit einem Messkreis gemessen. Eine Steuereinheit umfasst Einstelleinheiten und Vergleichseinheiten. Die Einstelleinheiten geben Referenzwerte vor. Die Vergleichseinheit vergleicht den Laserleistung-Messwert von dem Messkreis mit einem Referenzwert von der Einstelleinheit, während die Vergleichseinheit den Laserleistung-Messwert von dem Messkreis mit Referenzwerten von den Einstelleinheit vergleicht.

Das Dokument EP 2 109 197 A1 beschreibt einen Laseroszillator einer Laserbearbeitungsvorrichtung. Der Laseroszillator umfasst einen optischen Resonator, der aus zwei Begrenzungsspiegeln gebildet wird und innerhalb seines optischen Strahlengangs ein aktives Medium, einen nichtlinearen Kristall und einen Separator-Auslass-Spiegel umfasst. Im aktiven Medium wird ein Strahl einer Grundwelle erzeugt, der zwischen den beiden Begrenzungsspiegeln eingeschlossen ist. Der nichtlineare Kristall erzeugt einen Strahl einer zweiten Harmonischen im Strahlengang des optischen Resonators als Folge eines nichtlinearen Prozesses zwischen dem nichtlinearen Kristall und dem Strahl der Grundwelle. Der Strahl der zweiten Harmonischen wird mit Hilfe des Separator-Auslass-Spiegels aus dem Strahlengang des optischen Resonators als ein Auslassstrahl separiert, der auf ein Werkstück strahlt. Ein Laser-Auslass-Messkreis erzeugt ein elektrisches Signal, das für die Laser-Auslass des gepulsten Laserstrahls der zweiten Harmonischen repräsentativ ist. Eine Steuereinheit vergleicht dieses Signal mit einem Referenzwert von einer Einstelleinheit.

Aufgabe der Erfindung ist es, einen Lösungsweg anzugeben, um in einem für die Materialbearbeitung dienenden Lasersystem die Strahlungsqualität der Laserstrahlung überwachen zu können. Eine solche Überwachung soll es ermöglichen, den Bearbeitungsprozess bei Verschlechterung der Strahlungsqualität zu unterbrechen oder anderweitig zu reagieren. Insbesondere soll die Überwachung in Echtzeit, d.h. während der Materialbearbeitung, möglich sein. Die Strahlungsqualität bezieht sich hierbei insbesondere auf die Fokussierbarkeit sowie den zeitlichen Verlauf der Impulseinhüllenden (zeitliche Einhüllende des einzelnen Laserpulses), d.h. die Kürze der Impulsdauer. Die Fokussierbarkeit wird maßgeblich durch die Qualität des Strahlprofils und der Wellenfront der Laserstrahlung bestimmt.

Diese Aufgabe wird durch eine Materialbearbeitungseinrichtung mit den Merkmalen des Anspruchs 1 und durch ein Verfahren zum Betreiben eines solchen mit den Merkmalen des Anspruchs 11 gelöst.

Die erfindungsgemäße Einrichtung dient zur Bearbeitung eines Materials mit einem gepulsten Laserstrahl und umfasst:
- einen Laser zur Bereitstellung des Laserstrahls,
- eine Messanordnung, um Messwerte für eine Grundwellenleistung des Laserstrahls sowie für eine Leistung mindestens einer durch Frequenzvervielfachung aus dem Laserstrahl erzeugten höheren Harmonischen zu gewinnen,
- eine mit der Messanordnung verbundene Auswerteeinheit, welche dazu eingerichtet ist, die Qualität des Laserstrahls abhängig von der gemessenen Grundwellenleistung, der gemessenen Leistung der höheren Harmonischen und einer eingestellten Strahlungsleistung des Lasers zu untersuchen.

Die Erfindung lehrt, für die Erfassung der zeitlichen oder/und räumlichen Strahlungsqualität gepulster Laserstrahlung (insbesondere mit Femtosekunden-Pulsen) bzw. zur Erfassung von Veränderungen dieser Qualität Prozesse zu untersuchen, deren Effizienz von der Intensität der applizierten, fokussierten Laserstrahlung abhängt. Nichtlineare Frequenzmischungen wie die Frequenzverdopplung sind ein Beispiel für solche Prozesse. In einer zweckmäßigen Ausgestaltung wird dabei ein (kleiner) Teil der Laserstrahlung in einen optisch nichtlinearen Kristall fokussiert und in Strahlung der doppelten Frequenz umgewandelt. Nimmt man eine infrarote Wellenlänge der Laserstrahlung des Lasers an, so wird der in den Kristall fokussierte Strahlungsteil beispielsweise in grünes Licht umgewandelt. Da die Frequenzverdopplung quadratisch von der einfallenden Leistung abhängig, hängt die Effizienz dieses Prozesses von der applizierten Leistungsdichte (räumlich und zeitlich) im Kristall und damit von der Fokussierbarkeit und der Impulsdauer der Laserstrahlung ab. Somit lässt sich - soweit andere Einflussparameter ausgeschlossen werden können - eine Leistungsänderung des frequenzverdoppelten Lichts auf eine Änderung in der Fokussierbarkeit oder/und der Impulsdauer zurückführen. Eine solche Änderung kann beispielsweise durch eine Beeinträchtigung der Wellenfront der Laserstrahlung oder durch eine Pulsdauerverlängerung hervorgerufen werden.

Eine Erkenntnis der Erfindung ist es, dass es im Allgemeinen aufwendig und schwierig ist, all diejenigen Parameter, welche die Qualität des Fokus (d.h. die Fokussierbarkeit) nachhaltig beeinflussen können, gleichzeitig nebeneinander zu überwachen. Die erfindungsgemäße Methodik ermöglicht demgegenüber eine summarische Qualitätskontrolle in Echtzeit, d.h. sozusagen online. Sobald irgendeiner dieser die Fokuserzeugung beeinflussenden Parameter schlechter wird, kann dies über das Messsignal der höheren Harmonischen detektiert werden und es kann entsprechend reagiert werden. Für den Betrieb der Materialbearbeitungseinrichtung kann es als nebensächlich angesehen werden, welcher konkrete Parameter für eine beobachtete Verschlechterung der Fokussierbarkeit der Laserpulse ursächlich ist. Wenn die Fokussierbarkeit beeinträchtigt ist, empfiehlt es sich, ab Überschreiten bestimmter vorgegebener Grenzen schnellstmöglich die Bearbeitung abzubrechen. Jeden einzelnen Laserparameter, der für eine effektive Fokuserzeugung relevant ist und die Fokussierbarkeit der Laserstrahlung verschlechtern kann, gesondert zu überwachen, erforderte einen enorm hohen Aufwand. Es wäre auch ausgesprochen schwierig, eine instantane und zuverlässige Reaktion auf eine Degradation eines einzelnen dieser Parameter zu realisieren. Die durch die Erfindung gegebene Möglichkeit einer summarischen Online-Überwachung aller für die Fokussierbarkeit relevanten Laserparameter schafft demgegenüber einen beträchtlichen Vorteil, weil der Aufwand erheblich geringer wird.

Prinzipiell sind im Rahmen der Erfindung auch nichtlineare Prozesse höherer Ordnung einsetzbar; der Kerngedanke der Erfindung ist keineswegs auf eine Frequenzverdopplung beschränkt. Allerdings sind für Prozesse höherer Ordnung in der Regel höhere Strahlungsintensitäten erforderlich, welche die Komplexität der erfindungsgemäßen Lösung erhöhen können.

Die optische Frequenzverdopplung ist ein Spezialfall der 3-Wellen-Mischung, bei der für hohe Feldstärken aus zwei Grundwellen mit den Kreisfrequenzen ω1 und ω2 eine dritte Welle mit der Kreisfrequenz ω3 = ω1 ± ω2 entsteht (ω = 2πf). Dabei entspricht ω3 = ω1 + ω2 der Summenfrequenzmischung und ω3 = ω1 - ω2 der Differenzfrequenzmischung. Beträgt nun im Spezialfall der Frequenzverdopplung ω1 = ω2, so entsteht lediglich eine Summenfrequenz ω3 = 2ω1 = 2ω2.

In einer praktischen Ausgestaltung der Erfindung wird ein Teil der Laserstrahlung in ein optisch-nichtlineares Medium (z.B. Kristall) appliziert. Bevorzugt wird der applizierte Teil der Laserstrahlung in das nichtlineare Medium fokussiert. In Folge der hohen Intensität im Fokus werden Ladungsträgerschwingungen in dem nichtlinearen Medium angeregt, die auch Oberwellen verschiedener Ordnung der eingestrahlten Fundamentalschwingung (Grundwelle) enthalten. Die zweite Harmonische stellt dabei die frequenzverdoppelte Welle mit der doppelten Frequenz und der halben Wellenlänge der Grundwelle dar. Durch geeignete Wahl der Einstrahlrichtung in das nichtlineare Medium bzw. durch geeignete Orientierung des nichtlinearen Mediums zur Strahlausbreitungsrichtung kann die Erzeugung der zweiten Harmonischen gegenüber den anderen Frequenzen bevorzugt werden. Zur ausführlichen theoretischen Beschreibung dieses Prozesses wird auf die einschlägige Fachliteratur verwiesen; auf eine nähere Erläuterung kann an dieser Stelle verzichtet werden.

Die instantane Intensität der zweiten Harmonischen ist bei dem obigen Prozess proportional zum Quadrat der instantanen Intensität der Grundwelle. Maßgeblich wird die Höhe des Signals der zweiten Harmonischen dabei durch die Spitzenintensität der Grundwelle bestimmt. Diese hängt ihrerseits von der Pulsenergie, von der Spotgröße (quadratischer Taillenradius) der Grundwelle im Fokus sowie der Pulsdauer der Grundwellenstrahlung ab. Nachdem die Effizienz der Frequenzverdopplung (Konversionseffizienz) - definiert als Quotient der zeitlich gemittelten Leistung der zweiten Harmonischen zur zeitlich gemittelten Leistung der Grundwellenstrahlung - gleichermaßen proportional zur Grundwellenleistung und damit zur Spitzenintensität der Grundwellenstrahlung ist, gilt auch für die Konversionseffizienz eine Abhängigkeit von der Spotgröße der Grundwellenstrahlung im Fokuspunkt und von ihrer Pulsdauer. Bei gleichbleibender Pulsenergie der Grundwellenstrahlung lässt somit eine Veränderung der Konversionseffizienz auf eine Änderung der zeitlichen oder/und räumlichen Strahlqualität (Pulseinhüllende, Strahlprofil, etc.) oder/und eine Änderung der Wellenfrontform und damit eine Änderung der Fokussierbarkeit schließen.

In einer bevorzugten Ausgestaltung der Erfindung ist die Länge eines als optisch-nichtlineares Medium verwendeten Kristalls signifikant größer als die Rayleigh-Länge der in den Kristall fokussierten Grundwellenstrahlung. Geringfügige Veränderungen der Divergenz der Grundwellenstrahlung (geringe Verschiebung der Fokusebene im Verdopplerkristall) wirken sich dann kaum auf das Intensitätssignal der zweiten Harmonischen aus.

Außerdem wird die Grundwellenstrahlung vorzugsweise vergleichsweise kurzbrennweitig in den Kristall fokussiert, um die Abhängigkeit der Phasenanpassung von der Richtung und spektralen Verteilung der in den Kristall applizierten Leistung zu mindern. Durch Fokussierung der applizierten Grundwellenstrahlung gelingt es, dass selbst im Fall einer leichten Veränderung der Einstrahlrichtung der Grundwellenstrahlung oder der Mittelwellenlänge der Grundwellenstrahlung stets geeignete Strahlungsanteile vorhanden sind, für die Phasenanpassung besteht. Es kann so ein vergleichsweise großer Winkelbereich der Einstrahlrichtung erhalten werden, für den ein zumindest näherungsweise gleiches Intensitätssignal der zweiten Harmonischen erhalten werden kann. Solange die Laserquelle innerhalb ihrer Spezifikationen arbeitet, beeinflusst sie demnach das Messverfahren nicht oder zumindest nur gering.

Wenn sich dagegen die Impulsdauer oder/und die Fokussierbarkeit beispielsweise in Folge von Wellenfrontverzerrungen der Grundwellenstrahlung verändert, wird dieser Effekt nicht kompensiert; stattdessen wirkt er sich direkt auf die Konversionseffizienz aus. Mit einer in den Strahlengang der Grundwellenstrahlung eingefügten verunreinigten Scheibe können sich beispielsweise Wellenfrontverzerrungen simulieren lassen, bei denen die Grundwellenleistung unter Umständen größtenteils oder vollständig erhalten bleibt. Da keine ebenen Wellenflächen mehr erkennbar sind, lässt sich dann kein guter Fokus mehr erzielen, weswegen die Frequenzverdopplung massiv geschwächt wird oder sogar zum Erliegen kommt. Die Veränderung der Leistung der zweiten Harmonischen hängt demnach direkt mit einer Veränderung der Wellenfront der Grundwellenstrahlung zusammen. Wellenfrontverzerrungen im Lasersystem können deshalb durch Überwachung der Leistung der zweiten Harmonischen (oder einer anderen, höheren Harmonischen) und durch Vergleich dieser Leistung mit einer Referenzleistung detektiert werden, welche vorzugsweise repräsentativ ist für diejenige Leistung der zweiten Harmonischen, die bei gegebener Grundwellenleistung bestenfalls erzielbar ist. Eine verschlechterte Strahlqualität der Grundwellenstrahlung ohne wesentlichen Leistungsverlust kann auf diese Weise sehr sensibel und instantan, d.h. in Echtzeit, erkannt werden und es kann entsprechend rasch reagiert werden, bevor größere Leistungsabfälle detektierbar sind.

Eine beispielhafte Vorgehensweise, um in Echtzeit die Strahlqualität eines von einem Lasersystem bereitgestellten Bearbeitungs-Laserstrahls zu überwachen, kann darin bestehen, dass zunächst ein Kalibrierungslauf durchgeführt wird, bei dem der Laser auf verschiedene (nominelle) Strahlungsleistungen innerhalb seines möglichen Leistungsbereichs eingestellt wird und bei jeder eingestellten Strahlungsleistung die Grundwellenleistung sowie die Leistung der zweiten (oder einer anderen höheren) Harmonischen gemessen wird. Der Kalibrierungslauf sollte dabei in einem störungsfreien Zustand des Lasersystems bei bester Fokussierbarkeit der Grundwellenstrahlung durchgeführt werden. Auf diese Weise kann erreicht werden, dass die Messwerte für die Grundwellenleistung sowie für die Leistung der höheren Harmonischen Maximal- bzw. Optimalwerte darstellen. Diese Maximalwerte repräsentieren die bei der jeweils eingestellten Strahlungsleistung des Lasers maximal erreichbaren Leistungen der Grundwelle und der höheren Harmonischen.

Durch Quotientenbildung (Leistung der zweiten Harmonischen dividiert durch Grundwellenleistung) kann zudem noch in Zuordnung zu jeder eingestellten Strahlungsleistung des Lasers ein Wert für die Konversionseffizienz ermittelt werden.

Die im Rahmen des Kalibrierungslaufs gewonnen Daten können beispielsweise in tabellarischer Form in einem Speicher abgespeichert werden.

Während der anschließenden Materialbearbeitung werden kontinuierlich oder in zeitlichen Abständen wiederum die Grundwellenleistung sowie die Leistung der höheren Harmonischen gemessen. Es können sodann zwei separate Prüfschritte durchgeführt werden. In einem ersten Prüfschritt kann die gemessene Grundwellenleistung mit einem Referenzwert verglichen werden. Dieser Referenzwert wird aus den im Speicher gespeicherten, im Rahmen des Kalibrierungslaufs gewonnenen Daten ermittelt. Er repräsentiert den optimalen Wert der Grundwellenleistung bei der eingestellten Strahlungsleistung des Lasers. Weicht die gemessene Grundwellenleistung nicht wesentlich von dem Referenzwert ab oder liegt sie innerhalb vorgegebener zulässiger Grenzen in Bezug auf den Referenzwert, bedeutet dies, dass zumindest keine Störungen vorliegen, welche die Leistung des Laserstrahls signifikant beeinträchtigen können. Liegt jedoch eine Abweichung vor, die größer als eine vorgegebene zulässige Grenze ist, kann dies zum Anlass genommen werden, eine vorbestimmte Warn- oder Notreaktion auszulösen, die beispielsweise die Ausgabe eines Warnhinweises oder/und eine Abschaltung des Lasers beinhalten kann.

In einem zweiten Prüfschritt kann zudem die gemessene Leistung der höheren Harmonischen mit einem Referenzwert verglichen werden oder/und es kann die Konversionseffizienz (Quotient aus gemessener Leistung der höheren Harmonischen und gemessener Grundwellenleistung) mit einem Referenzwert verglichen werden. Auch die Referenzwerte für die Leistung der höheren Harmonischen bzw. die Konversionseffizienz werden aus den gespeicherten Kalibrierungsdaten ermittelt; sie geben Sollwerte (Optimalwerte) für die Leistung der höheren Harmonischen bzw. die Konversionseffizienz bei der nominell eingestellten Strahlungsleistung des Lasers, d.h. im Arbeitspunkt des Lasers, an. Ergibt der Vergleich eine Abweichung über vorgegebene Grenzen hinaus, kann eine Warn- oder Notreaktion ausgelöst werden, wie zuvor erläutert. Ergeben dagegen sowohl der erste als auch der zweite Prüfschritt (die selbstverständlich nicht notwendig in dieser Reihenfolge ausgeführt werden müssen) keine unzulässigen Abweichungen, kann die Abgabe des Laserstrahls gestattet werden und der Betrieb der Materialbearbeitungseinrichtung kann ungehindert fortgesetzt werden.

Mit dem zweiten Prüfschritt gelingt es insbesondere, solche Störungen zu detektieren, die keine oder nur eine geringe Leistungsabschwächung des Laserstrahls bewirken, aber signifikante Wellenfrontverzerrungen hervorrufen. Im Fall von unerwünschten Wellenfrontstörungen kann das Verhältnis von gemessener zu maximaler Leistung der zweiten Harmonischen ohne weiteres auf wenige Prozent (beispielsweise unter 15 Prozent) sinken, was zeigt, dass die Leistung der zweiten Harmonischen ein guter Gradmesser für die Strahlqualität eines Laserstrahls ist.

Gemäß bevorzugter Weiterbildungen der erfindungsgemäßen Materialbearbeitungseinrichtung kann die mindestens eine höhere Harmonische die zweite Harmonische umfassen, und die Pulsdauer des Laserstrahls liegt vorzugsweise im Femtosekundenbereich.

Die Auswerteeinheit kann dazu eingerichtet sein, mindestens einen der Leistungsmesswerte oder/und einen hieraus abgeleiteten Wert (beispielsweise Konversionseffizienz) mit mindestens einem Referenzwert zu vergleichen und abhängig vom Maß einer Abweichung des Messwerts bzw. des abgeleiteten Werts von dem Referenzwert eine vorbestimmte Reaktion zu bewirken. Dabei kann der Referenzwert einen bei der eingestellten Strahlungsleistung des Lasers maximal erzielbaren Wert für die Grundwellenleistung, die Leistung der höheren Harmonischen oder eine Konversionseffizienz der Frequenzvervielfachung repräsentieren.

Die Auswerteeinheit kann ferner dazu eingerichtet sein, den Referenzwert vorab gespeicherten Referenzinformationen zu entnehmen, welche in Zuordnung zu einer Vielzahl unterschiedlicher eingestellter Strahlungsleistungen des Lasers Referenzwerte für die Grundwellenleistung oder/und die Leistung der höheren Harmonischen oder/und eine Konversionseffizienz der Frequenzvervielfachung repräsentieren. Alternativ oder zusätzlich kann die Auswerteeinheit dazu eingerichtet sein, einen Quotienten aus der gemessenen Leistung der höheren Harmonischen und der gemessenen Grundwellenleistung oder/und einen Quotienten aus der gemessenen Leistung der höheren Harmonischen und einem Referenzwert für die Leistung der höheren Harmonischen zu ermitteln und die Strahlqualität abhängig von dem ermittelten Quotienten zu untersuchen.

Die Auswerteeinheit kann zudem Teil einer elektronischen Steueranordnung sein, welche dazu eingerichtet ist, abhängig vom Maß der Abweichung des Messwerts bzw. des abgeleiteten Werts von dem Referenzwert die Abgabe des Laserstrahls zu steuern. Beispielsweise kann sie die Abgabe des Laserstrahls zulassen, falls die ermittelte Abweichung innerhalb vorbestimmter Grenzen liegt, oder/und sie kann die Abgabe des Laserstrahls unterbinden, falls die ermittelte Abweichung außerhalb solcher Grenzen liegt.

Die Messanordnung umfasst zur Erzeugung der höheren Harmonischen ein optisch nichtlineares Medium sowie eine diesem Medium vorgeschaltete Fokussiereinheit zur Fokussierung eines aus dem Laserstrahl ausgekoppelten Teilstrahls auf das nichtlineare Medium. Sie kann ferner dazu eingerichtet sein, als Grundwellenleistung die Leistung eines aus dem Laserstrahl ausgekoppelten Teilstrahls zu messen.

Die Auswerteeinheit kann Teil einer elektronischen Steueranordnung sein, welche dazu eingerichtet ist, in einem Kalibrierprozess nacheinander eine Vielzahl unterschiedlicher Strahlungsleistungen des Lasers einzustellen, in Zuordnung zu jeder eingestellten Strahlungsleistung des Lasers Messwerte für die Grundwellenleistung und die Leistung der höheren Harmonischen zu ermitteln und die ermittelten Messwerte oder/und hiervon abgeleitete Werte in einem Speicher in Zuordnung zu den verschiedenen Strahlungsleistungen des Lasers abzuspeichern.

Das erfindungsgemäße Verfahren zum Betreiben einer mit einem gepulsten Laserstrahl arbeitenden Materialbearbeitungseinrichtung umfasst:
- Messen einer Grundwellenleistung des Laserstrahls und einer Leistung mindestens einer durch Frequenzvervielfachung aus dem Laserstrahl erzeugten höheren Harmonischen,
- Vergleichen mindestens eines der Leistungsmesswerte oder/und eines hieraus abgeleiteten Werts mit mindestens einem Referenzwert,
- Bewirken einer vorbestimmten Reaktion abhängig vom Maß einer Abweichung des Messwerts bzw. des abgeleiteten Werts von dem Referenzwert.

Das Verfahren kann ferner das vorherige Durchführen eines Kalibrierprozesses umfassen, bei dem Messwerte für die Grundwellenleistung und die Leistung der höheren Harmonischen bei einer Vielzahl unterschiedlicher eingestellter Strahlungsleistungen eines den Laserstrahl bereitstellenden Lasers gewonnen werden und die gewonnenen Messwerte oder/und hiervon abgeleitete Werte in einem Speicher in Zuordnung zu den verschiedenen Strahlungsleistungen des Lasers abgespeichert werden, wobei der Referenzwert aus den im Speicher gespeicherten Messwerte oder/und abgeleiteten Werten gewonnen wird.

Die vorbestimmte Reaktion kann eine optische oder/und akustische Meldung oder/und ein Freigeben oder ein Abschalten des Laserstrahls umfassen. Ferner können die Schritte des Messens und Vergleichens wiederholt, insbesondere in regelmäßigen zeitlichen Abständen, während der Bearbeitung eines Materials mit dem Laserstrahl durchgeführt werden. Auf diese Weise ist sozusagen eine Online-Überwachung in Echtzeit möglich, wobei die Überwachungsfrequenz vom Zeitintervall abhängt, mit dem die Messungen durchgeführt werden. Diesbezüglich gibt es an sich keine durch die Erfindung vorgegebenen Grenzen, d.h. es kann so oft gemessen werden, wie es die Messapparatur und die verfügbare Rechenleistung zulassen.

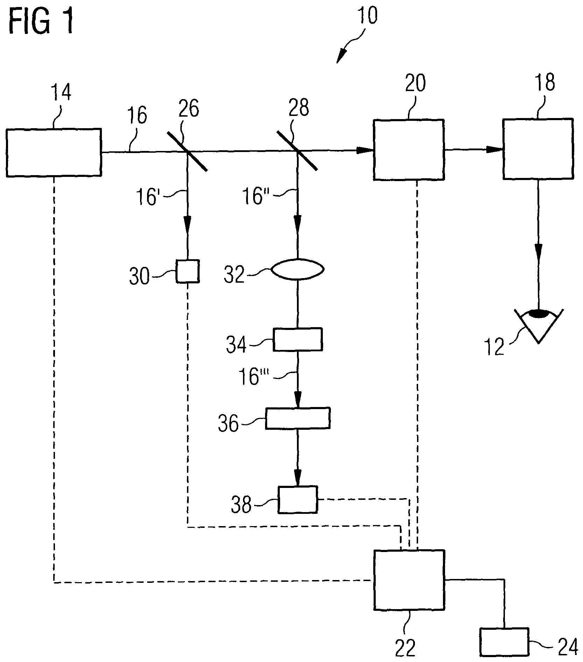

Die Erfindung wird nachfolgend anhand der beigefügten einzigen Zeichnung näher erläutert. Deren Figur 1 zeigt schematisch ein Ausführungsbeispiel einer Materialbearbeitungseinrichtung zur Laserbearbeitung eines Materials. Die allgemein mit 10 bezeichnete Materialbearbeitungseinrichtung dient im gezeigten Beispielfall zur laserchirurgischen Bearbeitung eines humanen Auges 12 und dort beispielsweise zur Anbringung intrakornealer Gewebeschnitte. Sie umfasst eine Laserquelle 14, welche einen gepulsten Laserstrahl 16 mit Pulsdauern im Femtosekundenbereich erzeugt. Die Laserquelle 14 umfasst beispielsweise einen Faserlaser. Des Weiteren umfasst die Materialbearbeitungseinrichtung 10 eine Fokussieroptik 18 zur Fokussierung des Laserstrahls 16 auf das zu bearbeitende Objekt, hier das Auge 12. Die Fokussieroptik 18 ist beispielsweise von einem F-Theta-Objektiv gebildet. Der Fokussieroptik 18 vorgeschaltet ist ein Scanner 20, welcher der transversalen und longitudinalen Steuerung der Fokusposition des Laserstrahls 16 dient. Zur transversalen Ablenkung kann der Scanner 20 beispielsweise ein galvanometrisch gesteuertes Spiegelpaar oder einen elektrisch gesteuerten Ablenkkristall umfassen. Für die longitudinale Fokussteuerung kann der Scanner 20 beispielsweise ein die Divergenz des Laserstrahls 16 beeinflussendes optisches Element umfassen, etwa eine in Strahlausbreitungsrichtung längsverschiebliche Linse oder eine Flüssiglinse variabler Brechkraft oder einen deformierbaren Spiegel. Es versteht sich, dass die für die transversale Fokussteuerung und die für die longitudinale Fokussteuerung des Laserstrahls 16 verantwortlichen Komponenten des Scanners 20 an unterschiedlichen Stellen entlang der Ausbreitungsrichtung des Laserstrahls 16 angeordnet sein können. Der Scanner 20 muss dementsprechend keine kompakte Einheit sein; es kann sich um eine verteilte Anordnung verschiedener Scan-Komponenten handeln.

Zur Steuerung der Laserquelle 14 und des Scanners 20 ist eine mikroprozessorgestützte Steuereinheit 22 vorgesehen, welche nach Maßgabe eines in einem Speicher 24 gespeicherten Steuerprogramms arbeitet. Das Steuerprogramm enthält geeignete Steuerparameter (beispielsweise in Form von Koordinaten für die einzelnen Schusspositionen der Laserpulse), welche die zu erzeugende Schnittgeometrie festlegen.

Für die Erzeugung feiner und präziser Schnitte mittels des Laserstrahls 16 ist eine hohe räumliche und zeitliche Strahlqualität desselben wünschenswert. Zur Echtzeit-Überwachung der Strahlqualität des Laserstrahls 16 weist die Materialbearbeitungseinrichtung 10 Mittel auf, um in Strahlausbreitungsrichtung vor der transversalen Scanner-Ablenkung aus dem Laserstrahl 16 zwei Teilstrahlen 16', 16" auszukoppeln. Im gezeigten Beispielfall umfassen diese Mittel zwei im Strahlengang des Laserstrahls 16 hintereinander angeordnete halbdurchlässige Teilerspiegel 26, 28. Ein erster Leistungsmesser 30 dient zur Messung der (zeitlich gemittelten) Strahlungsleistung des Teilstrahls 16'. Der Leistungsmesser 30 umfasst hierzu beispielsweise eine Photodiode und liefert ein für die gemessene Strahlungsleistung repräsentatives, insbesondere hierzu proportionales Signal an die Steuereinheit 22. Die von dem Leistungsmesser 30 gemessene Leistung entspricht einer Grundwellenleistung im Sinne der Erfindung.

Der zweite Teilstrahl 16" wird mittels einer Fokussierlinse 32 auf einen optischnichtlinearen Kristall 34 (oder ein anderes nichtlineares Medium) fokussiert, in dem durch nichtlineare Prozesse ein frequenzverdoppelter Strahl 16'" erzeugt wird, der anschließend durch ein optisches Bandpassfilter 36 von etwaigen störenden Nebenlinien sowie Resten der Grundwelle befreit wird. Ein zweiter Leistungsmesser 38, der wiederum beispielsweise eine Photodiode zur Strahlungsdetektion enthalten kann, dient zur Messung der (zeitlich gemittelten) Leistung des frequenzverdoppelten Strahls 16'" und liefert ein für die gemessene Leistung repräsentatives Signal an die Steuereinheit 22. Die gemessene Leistung des frequenzverdoppelten Strahls 16'" stellt eine Leistung einer aus der Grundwelle erzeugten höheren (hier zweiten) Harmonischen im Sinne der Erfindung dar.

Die Wellenlänge des von der Laserquelle 14 erzeugten Laserstrahls 16 liegt beispielsweise im infraroten Bereich zwischen 1000 und 1100 nm. Es entsteht so durch Frequenzverdopplung in dem nichtlinearen Kristall 34 ein grüner bis gelblich-grüner Lichtstrahl 16"'.

Die Steuereinheit 22 berechnet aus den von den Leistungsmessern 30, 38 gelieferten Leistungsmesssignalen eine Konversionseffizienz, indem sie die gemessene Leistung des Lichtstrahls 16"' durch die gemessene Leistung des Teilstrahls 16' dividiert.

In dem Speicher 24 sind zusätzlich Kalibrierungsdaten abgelegt, welche von der Steuereinheit 22 in einem vorangehenden Kalibrierungsprozess ermittelt wurden. Die Kalibrierungsdaten geben für unterschiedliche nominelle Werte der Strahlungsleistung des Lasers 14 jeweils einen Referenzwert für die Leistung des Teilstrahls 16', für die Leistung des frequenzverdoppelten Strahls 16'" sowie für die Konversionseffizienz an. Die unterschiedlichen Strahlungsleistungen des Lasers 14 entsprechen unterschiedlichen Arbeitspunkten. Der Laser 14 ist in einem Bereich von Strahlungsleistungen betreibbar; je nach Anwendungsfall kann die Steuereinheit 22 einen unterschiedlichen Arbeitspunkt des Lasers 14 einstellen. Beispielsweise sind die verschiedenen Strahlungsleistungen durch prozentuale Werte repräsentiert, welche den jeweiligen Arbeitspunkt durch eine auf eine maximal einstellbare Strahlungsleistung des Lasers 14 bezogene Prozentangabe definieren. Die gespeicherten Referenzwerte beziehen sich jeweils auf eine optimale Situation, d.h. einen störungsfreien Betrieb ohne (zumindest ohne wesentliche) unerwünschte Wellenfrontverzerrungen des Laserstrahls 16 und ohne (zumindest ohne wesentliche) unerwünschte Leistungsabschwächung des Laserstrahls 16 längs dessen Ausbreitungsweg. Sie stellen dementsprechend bestmöglich erreichbare Werte für die Leistung des Teilstrahls 16', die Leistung des frequenzverdoppelten Strahls 16'" und die Konversionseffizienz dar.

Die aktuell gemessenen Leistungswerte für den Teilstrahl 16' und den frequenzverdoppelten Strahl 16"' werden von der Steuereinheit 22 verwendet, um anhand eines Vergleichs mit den im Speicher 24 gespeicherten Kalibrierungsdaten die Strahlqualität des Laserstrahls 16 zu untersuchen. Konkret prüft die Steuereinheit 22, ob die gemessene Leistung des Teilstrahls 16' zumindest näherungsweise demjenigen Referenzwert entspricht, der im Speicher 24 für die Grundwellenleistung bei dem betreffenden Arbeitspunkt des Lasers 14 abgespeichert ist. Auf diese Weise kann die Steuereinheit 22 unerwünschte Leistungsabschwächungen des Laserstrahls 16 gegenüber dem störungsfreien Fall detektieren.

Ferner prüft die Steuereinheit 22, ob die gemessene Leistung des frequenzverdoppelten Strahls 16"' demjenigen Referenzwert zumindest näherungsweise entspricht, der in den gespeicherten Kalibrierungsdaten für die Leistung der zweiten Harmonischen bei dem betreffenden Arbeitspunkt des Lasers 14 enthalten ist. Alternativ oder zusätzlich prüft die Steuereinheit 22, ob die berechnete Konversionseffizienz (berechnet als Quotient der gemessenen Leistung des Lichtstrahls 16"' und der gemessenen Leistung des Teilstrahls 16') zumindest näherungsweise demjenigen Referenzwert entspricht, der in den Kalibrierungsdaten für die Konversionseffizienz bei dem betreffenden Arbeitspunkt des Lasers 14 enthalten ist. Die Prüfung der gemessenen Leistungswerte bzw. der hieraus abgeleiteten Konversionseffizienz auf Entsprechung zu den gespeicherten Referenzwerten kann beispielsweise eine Differenzbildung oder/und eine Quotientenbildung umfassen. Nachdem die Referenzwerte Optimalwerte angeben, die im störungsfreien Betrieb der Materialbearbeitungseinrichtung 10 bei optimaler Fokussierbarkeit des Laserstrahls 16 erzielbar sind, sind etwaige Abweichungen zwischen den gemessenen Leistungen bzw. der berechneten Konversionseffizienz von den Referenzwerten ein Indikator für unerwünschte Leistungsabschwächungen oder/und unerwünschte Wellenfrontverzerrungen oder/und unerwünschte Pulsdauerverlängerungen des Laserstrahls 16. Überschreiten die ermittelten Abweichungen ein vorgegebenes Maß, kann die Steuereinheit 22 die Abgabe des Laserstrahls 16 unterbrechen.

Der erwähnte Kalibrierungsprozess kann von der Steuereinheit 22 automatisch durchgeführt werden, beispielsweise jedes Mal wenn die Materialbearbeitungseinrichtung 10 eingeschaltet wird oder wenn ein Anwender einen geeigneten Startbefehl zur Durchführung der Kalibrierung eingibt. Im Rahmen des automatischen Kalibrierungsprozesses kann die Steuereinheit 22 nacheinander unterschiedliche Arbeitspunkte des Lasers 14 einstellen und die jeweils ermittelten Messwerte für die Grundwellenleistung und die Leistung der zweiten Harmonischen im Speicher 24 abspeichern.

Zur Fokussierung des zweiten Teilstrahls 16" kann statt der Linse 32 beispielsweise auch ein diffraktives optisches Element verwendet werden. Das nichtlineare Medium 34 kann beispielsweise ein periodisch gepolter Kristall sein. Der Kristall kann temperaturstabilisiert sein, wobei die Phasenanpassung über die Temperatur optimiert werden kann. Darüber hinaus ist es möglich, gemäß der in der Fachwelt an sich bekannten Methode der nicht-kritischen Phasenanpassung zu arbeiten.

## Patentansprüche

1. Einrichtung zur Bearbeitung eines Materials mit einem gepulsten Laserstrahl (16), umfassend
- einen Laser (14) zur Bereitstellung des Laserstrahls (16),
- eine Messanordnung (30, 32, 34, 36, 38), um Messwerte für eine Grundwellenleistung des Laserstrahls sowie für eine Leistung mindestens einer durch Frequenzvervielfachung aus dem Laserstrahl erzeugten höheren Harmonischen zu gewinnen, wobei die Messanordnung zur Erzeugung der höheren Harmonischen ein optisch nichtlineares Medium (34) sowie eine diesem Medium vorgeschaltete Fokussiereinheit (32) zur Fokussierung eines aus dem Laserstrahl (16) ausgekoppelten Teilstrahls (16") auf das nichtlineare Medium umfasst, **gekennzeichnet dadurch, dass** sie weiterhin umfasst
- eine mit der Messanordnung verbundene Auswerteeinheit (22), welche dazu eingerichtet ist, die Qualität des Laserstrahls (16) abhängig von der gemessenen Grundwellenleistung, der gemessenen Leistung der höheren Harmonischen und einer eingestellten Strahlungsleistung des Lasers (14) zu untersuchen.

2. Einrichtung nach Anspruch 1, wobei die mindestens eine höhere Harmonische die zweite Harmonische umfasst.

3. Einrichtung nach Anspruch 1 oder 2, wobei die Auswerteeinheit (22) dazu eingerichtet ist, mindestens einen der Leistungsmesswerte oder/und einen hieraus abgeleiteten Wert mit mindestens einem Referenzwert zu vergleichen und abhängig vom Maß einer Abweichung des Messwerts bzw. des abgeleiteten Werts von dem Referenzwert eine vorbestimmte Reaktion zu bewirken.

4. Einrichtung nach Anspruch 3, wobei der Referenzwert einen bei der eingestellten Strahlungsleistung des Lasers (14) maximal erzielbaren Wert für die Grundwellenleistung, die Leistung der höheren Harmonischen oder eine Konversionseffizienz der Frequenzvervielfachung repräsentiert.

5. Einrichtung nach Anspruch 3 oder 4, wobei die Auswerteeinheit (22) dazu eingerichtet ist, den Referenzwert vorab gespeicherten Referenzinformationen zu entnehmen, welche in Zuordnung zu einer Vielzahl unterschiedlicher eingestellter Strahlungsleistungen des Lasers (14) Referenzwerte für die Grundwellenleistung oder/und die Leistung der höheren Harmonischen oder/und eine Konversionseffizienz der Frequenzvervielfachung repräsentieren.

6. Einrichtung nach einem der Ansprüche 3 bis 5, wobei die Auswerteeinheit (22) dazu eingerichtet ist, einen Quotienten aus der gemessenen Leistung der höheren Harmonischen und der gemessenen Grundwellenleistung oder/und einen Quotienten aus der gemessenen Leistung der höheren Harmonischen und einem Referenzwert für die Leistung der höheren Harmonischen zu ermitteln und die Strahlqualität abhängig von dem ermittelten Quotienten zu untersuchen.

7. Einrichtung nach einem der Ansprüche 3 bis 6, wobei die Auswerteeinheit (22) Teil einer elektronischen Steueranordnung ist, welche dazu eingerichtet ist, abhängig vom Maß der Abweichung des Messwerts bzw. des abgeleiteten Werts von dem Referenzwert die Abgabe des Laserstrahls zu steuern.

8. Einrichtung nach einem der vorhergehenden Ansprüche, wobei die Messanordnung dazu eingerichtet ist, als Grundwellenleistung die Leistung eines aus dem Laserstrahl (16) ausgekoppelten Teilstrahls (16') zu messen.

9. Einrichtung nach einem der Ansprüche 3 bis 8, wobei die Auswerteeinheit (22) Teil einer elektronischen Steueranordnung ist, welche dazu eingerichtet ist, in einem Kalibrierprozess nacheinander eine Vielzahl unterschiedlicher Strahlungsleistungen des Lasers (14) einzustellen, in Zuordnung zu jeder eingestellten Strahlungsleistung des Lasers (14) Messwerte für die Grundwellenleistung und die Leistung der höheren Harmonischen zu ermitteln und die ermittelten Messwerte oder/und hiervon abgeleitete Werte in einem Speicher (24) in Zuordnung zu den verschiedenen Strahlungsleistungen des Lasers (14) abzuspeichern.

10. Einrichtung nach einem der vorhergehenden Ansprüche, wobei die Pulsdauer des Laserstrahls (16) im Femtosekundenbereich liegt.

11. Verfahren zum Betreiben einer Einrichtung zur Bearbeitung eines Materials mit einem gepulsten Laserstrahl (16), umfassend
- Messen einer Grundwellenleistung des Laserstrahls (16) und einer Leistung mindestens einer durch Frequenzvervielfachung aus dem Laserstrahl erzeugten höheren Harmonischen, wobei zur Erzeugung der höheren Harmonischen ein Teilstrahl (16") aus dem Laserstrahl (16) ausgekoppelt wird und mittels einer Fokussiereinheit (32) auf ein optisch nichtlineares Medium (34) fokussiert wird,
- **dadurch gekennzeichnet, dass** die Qualität des Laserstrahls (16) durch vergleichen mindestens eines der Leistungsmesswerte oder/und eines hieraus abgeleiteten Werts mit mindestens einem Referenzwert untersucht wird, und durch
- Bewirken einer vorbestimmten Reaktion abhängig vom Maß einer Abweichung des Messwerts bzw. des abgeleiteten Werts von dem Referenzwert.

12. Verfahren nach Anspruch 11, ferner umfassend das vorherige Durchführen eines Kalibrierprozesses, bei dem Messwerte für die Grundwellenleistung und die Leistung der höheren Harmonischen bei einer Vielzahl unterschiedlicher eingestellter Strahlungsleistungen eines den Laserstrahl bereitstellenden Lasers (14) gewonnen werden und die gewonnenen Messwerte oder/und hiervon abgeleitete Werte in einem Speicher (24) in Zuordnung zu den verschiedenen Strahlungsleistungen des Lasers (14) abgespeichert werden, wobei der Referenzwert aus den im Speicher gespeicherten Messwerten oder/und abgeleiteten Werten gewonnen wird.

13. Verfahren nach Anspruch 11 oder 12, wobei die vorbestimmte Reaktion eine optische oder/und akustische Meldung oder/und ein Abschalten oder eine Freigabe des Laserstrahls umfasst.

14. Verfahren nach einem der Ansprüche 11 bis 13, wobei die Schritte des Messens und Vergleichens wiederholt, insbesondere in regelmäßigen zeitlichen Abständen, während der Bearbeitung eines Materials mit dem Laserstrahl durchgeführt werden.

## Claims

1. Device for processing a material with a pulsed laser beam (16), comprising
- a laser (14) for providing the laser beam (16),
- a measuring arrangement (30, 32, 34, 36, 38) for obtaining measurement values for a fundamental wave power of the laser beam and for a power of at least one higher harmonic generated from the laser beam by frequency multiplication, wherein the measuring arrangement comprises an optical non-linear medium (34) for generating the higher harmonics and a focusing unit (32), disposed upstream of the medium, for focusing a partial beam (16") decoupled from the laser beam (16) onto the non-linear medium,
**characterized in that** said device furthermore comprises
- an evaluation unit (22) connected to the measuring arrangement, said evaluation unit being configured to examine the quality of the laser beam (16) in a manner dependent on the measured fundamental wave power, the measured power of the higher harmonic and a set radiant power of the laser (14).

2. Device according to Claim 1, wherein the at least one higher harmonic comprises the second harmonic.

3. Device according to Claim 1 or 2, wherein the evaluation unit (22) is configured to compare at least one of the power measurement values and/or a value derived therefrom with at least one reference value and to effect a predetermined reaction depending on the extent of a deviation of the measured value or the derived value from the reference value.

4. Device according to Claim 3, wherein the reference value represents a maximum obtainable value for the fundamental wave power, the power of the higher harmonic or a conversion efficiency of the frequency multiplication at the set radiant power of the laser (14).

5. Device according to Claim 3 or 4, wherein the evaluation unit (22) is configured to gather the reference value from previously stored reference information, which represents, assigned to a multiplicity of different set radiant powers of the laser (14), reference values for the fundamental wave power and/or the power of the higher harmonics and/or conversion efficiency of the frequency multiplication.

6. Device according to one of Claims 3 to 5, wherein the evaluation unit (22) is configured to establish a quotient of the measured power of the higher harmonic and the measured fundamental wave power and/or a quotient of the measured power of the higher harmonic and a reference value for the power of the higher harmonic, and examine the beam quality in a manner dependent on the established quotient.

7. Device according to one of Claims 3 to 6, wherein the evaluation unit (22) is part of an electronic control arrangement which is configured to control the output of the laser beam depending on the extent of the deviation of the measurement value or of the derived value from the reference value.

8. Device according to one of the preceding claims, wherein the measuring arrangement is configured to measure the power of a partial beam (16') decoupled from the laser beam (16) as fundamental wave power.

9. Device according to one of Claims 3 to 8, wherein the evaluation unit (22) is part of an electronic control arrangement which is configured to set a multiplicity of different radiant powers of the laser (14) in succession during the calibration process, to establish, assigned to each set radiant power of the laser (14), measurement values for the fundamental wave power and the power of the higher harmonics and to store, assigned to the various radiant powers of the laser (14), the established measurement values and/or values derived therefrom in a memory (24).

10. Device according to one of the preceding claims, wherein the pulse duration of the laser beam (16) lies in the femtosecond range.

11. Method for operating a device for processing a material with a pulsed laser beam (16), comprising
- measuring a fundamental wave power of the laser beam (16) and a power of at least one higher harmonic generated from the laser beam by frequency multiplication, wherein, for the purposes of generating the higher harmonic, a sub-beam (16") is decoupled from the laser beam (16) and focussed on an optically non-linear medium (34) by means of the focusing unit (32),
- **characterized in that** the quality of the laser beam (16) is examined by comparing at least one of the power measurement values and/or a value derived therefrom with at least one reference value and by
- effecting a predetermined reaction in a manner dependent on the extent of a deviation of the measurement value or of the derived value from the reference value.

12. Method according to Claim 11, furthermore comprising the earlier implementation of a calibration process, in which measurement values for the fundamental wave power and the power of the higher harmonic are obtained for a multiplicity of different set radiant powers of a laser (14) providing the laser beam and the obtained measurement values and/or values derived therefrom are stored in a memory (24) in a manner assigned to the various radiant powers of the laser (14), wherein the reference value is obtained from the derived values and/or measurement values stored in the memory.

13. Method according to Claim 11 or 12, wherein the predetermined reaction comprises an optical and/or acoustic communication and/or a switching-off or a release of the laser beam.

14. Method according to one of Claims 11 to 13, wherein the steps of measuring and comparing are carried out repeatedly, in particular at regular time intervals, during the processing of a material with the laser beam.

## Revendications

1. Dispositif d'usinage d'un matériau avec un rayon laser (16) pulsé, comprenant
- un laser (14) servant à délivrer le rayon laser (16),
- un arrangement de mesure (30, 32, 34, 36, 38) pour obtenir des valeurs mesurées d'une puissance d'onde fondamentale du rayon laser ainsi que d'une puissance d'au moins une harmonique supérieure générée à partir du rayon laser par multiplication de la fréquence, l'arrangement de mesure comprenant, pour générer l'harmonique supérieure, un milieu (34) optiquement non linéaire ainsi qu'une unité de focalisation (32) placée en amont de ce milieu et servant à focaliser sur le milieu non linéaire un rayon partiel (16'') découplé du rayon laser (16),
**caractérisé en ce qu'**il comprend en outre
- une unité d'interprétation (22) reliée à l'arrangement de mesure, laquelle est conçue pour analyser la qualité du rayon laser (16) en fonction de la puissance d'onde fondamentale mesurée, de la puissance mesurée de l'harmonique supérieure et d'une puissance de rayonnement réglée du laser (14).

2. Dispositif selon la revendication 1, l'au moins une harmonique supérieure incluant la deuxième harmonique.

3. Dispositif selon la revendication 1 ou 2, l'unité d'interprétation (22) étant conçue pour comparer au moins l'une des valeurs mesurées de la puissance et/ou une valeur dérivée de celle-ci avec au moins une valeur de référence et provoquer une réaction prédéfinie en fonction de la valeur d'un écart de la valeur mesurée ou de la valeur dérivée par rapport à la valeur de référence.

4. Dispositif selon la revendication 3, la valeur de référence représentant une valeur maximale pouvant être atteinte pour la puissance d'onde fondamentale avec la puissance de rayonnement réglée du laser (14), la puissance de l'harmonique supérieure ou un rendement de conversion de la multiplication de fréquence.

5. Dispositif selon la revendication 3 ou 4, l'unité d'interprétation (22) étant conçue pour prélever de la valeur de référence des informations de référence préalablement enregistrées, lesquelles représentent, en association avec une pluralité de puissances de rayonnement différentes réglées du laser (14), des valeurs de référence pour la puissance d'onde fondamentale et/ou la puissance de l'harmonique supérieure et/ou un rendement de conversion de la multiplication de fréquence.

6. Dispositif selon l'une des revendications 3 à 5, l'unité d'interprétation (22) étant conçue pour déterminer un quotient de la puissance mesurée de l'harmonique supérieure et de la puissance d'onde fondamentale mesurée et/ou un quotient de la puissance mesurée de l'harmonique supérieure et d'une valeur de référence pour la puissance de l'harmonique supérieure et analyser la qualité du rayon en fonction du quotient déterminé.

7. Dispositif selon l'une des revendications 3 à 6, l'unité d'interprétation (22) faisant partie d'un arrangement de commande électronique qui est conçu pour commander la délivrance du rayon laser en fonction de la valeur de l'écart de la valeur mesurée ou de la valeur dérivée par rapport à la valeur de référence.

8. Dispositif selon l'une des revendications précédentes, l'arrangement de mesure étant conçu pour mesurer en tant que puissance d'onde fondamentale la puissance d'un rayon partiel (16') découplé du rayon laser (16).

9. Dispositif selon l'une des revendications 3 à 8, l'unité d'interprétation (22) faisant partie d'un arrangement de commande électronique qui est conçu pour régler successivement une pluralité de puissances de rayonnement différentes du laser (14) au cours d'un processus de calibrage, déterminer des valeurs mesurées pour la puissance d'onde fondamentale et la puissance de l'harmonique supérieure en association avec chaque puissance de rayonnement réglée du laser (14) et enregistrer dans une mémoire (24) les valeurs mesurées déterminées et/ou des valeurs qui en sont dérivées en association avec les différentes puissances de rayonnement du laser (14).

10. Dispositif selon l'une des revendications précédentes, la durée d'impulsion du rayon laser (16) se trouvant dans la plage des femtosecondes.

11. Procédé pour faire fonctionner un dispositif d'usinage d'un matériau avec un rayon laser (16) pulsé, comprenant
- mesure d'une puissance d'onde fondamentale du rayon laser (16) et d'une puissance d'au moins une harmonique supérieure générée à partir du rayon laser par multiplication de la fréquence, un rayon partiel (16") étant découplé du rayon laser (16) en vue de générer l'harmonique supérieure et focalisé sur un milieu (34) optiquement non linéaire au moyen d'une unité de focalisation (32),
- **caractérisé en ce que** la qualité du rayon laser (16) est analysée en comparant au moins l'une des valeurs mesurées de la puissance et/ou une valeur qui en est dérivée avec au moins une valeur de référence et en
- provoquant une réaction prédéfinie en fonction de la valeur d'un écart de la valeur mesurée ou de la valeur dérivée par rapport à la valeur de référence.

12. Procédé selon la revendication 11, comprenant en outre l'exécution préalable d'un processus de calibrage lors duquel sont obtenues des valeurs mesurées pour la puissance d'onde fondamentale et la puissance de l'harmonique supérieure avec une pluralité de puissances de rayonnement différentes réglées d'un laser (14) qui délivre le rayonnement laser, et les valeurs mesurées obtenues et/ou des valeurs qui en sont dérivées sont enregistrées dans une mémoire (24) en association avec les différentes puissances de rayonnement du laser (14), la valeur de référence étant obtenue à partir des valeurs mesurées et/ou des valeurs dérivées enregistrées dans la mémoire.

13. Procédé selon la revendication 11 ou 12, la réaction prédéfinie comprenant un message visuel et/ou sonore et/ou une déconnexion ou une libération du rayon laser.

14. Procédé selon l'une des revendications 11 à 13, les étapes de mesure et de comparaison étant exécutées de manière répétée, notamment à des intervalles de temps réguliers, pendant l'usinage d'un matériau avec le rayon laser.
